# EUROPÄISCHE PATENTSCHRIFT

(11) **EP 0 663 181 B1**
(45) Veröffentlichungstag und Bekanntmachung des Hinweises auf die Patenterteilung: **16.02.2000**
(21) Anmeldenummer: 94120639.3
(22) Anmeldetag: 24.12.1994
(51) Int. Cl.: A61B 5/107, A61B 5/103

(54) **Anzeigesystem zur Vermessung des menschlichen Körpers**
Display system for measurements of human bodies
Système d'affichage pour la mensuration des corps humains

(30) Priorität: 15.01.1994 DE 4401036
(43) Veröffentlichungstag der Anmeldung: 19.07.1995
(73) Patentinhaber: Otto Bock Orthopädische Industrie Besitz- und Verwaltungs-Kommanditgesellschaft, 37115 Duderstadt (DE)
(72) Erfinder: Blumentritt, Siegmar, Dr., D-37115 Duderstadt (DE); Brendel, Thomas, Dipl.-Ing., D-37115 Duderstadt (DE); Sawatzki, Steffen, Dipl.-Ing., D-99759 Sollstedt (DE); Van de Veen, Paul Gerad, Dr.Ir., NL-7514 EC Enschede (NL)
(74) Vertreter: Gramm, Werner, Prof. Dipl.-Ing.

(56) Entgegenhaltungen:
- EP-A- 0 119 660
- GB-A- 2 121 688
- NL-A- 7 415 910
- US-A- 4 832 049
- CLINICAL BIOMECHANICS, Bd. 7, Nr. 2, 1.Mai 1992, Seiten 80-86, XP000267544 PEARSALL D J ET AL: "LINE OF GRAVITY RELATIVE TO UPRIGHT VERTEBRAL POSTURE"

## Beschreibung

Die Erfindung betrifft ein schwerpunktgesteuertes Anzeigesystem zur Vermessung des menschlichen Körpers und/oder prothetischer und orthetischer Paßteile in ihrer relativen Position zum menschlichen Körper.

Eine hinsichtlich aller Aktivitäten eines Prothesenträgers optimal gestaltete Prothese oder Orthese ist nicht denkbar. So ist z.B. eine für das Gehen optimal ausgelegte Prothese in der Regel nicht optimal für das Sitzen des Prothesenträgers. Da jedoch das Gehen die wichtigste Funktion darstellt, wird man in der Regel versuchen, hierfür einen optimalen Prothesenaufbau zu ermitteln, um dann andere Funktionen, wie Stehen, Sitzen oder Liegen zu untersuchen, um gegebenenfalls Änderungen im Aufbau der Prothese vorzunehmen.

Bei der entsprechenden Vermessung des menschlichen Körpers spielt dessen Schwerpunktlinie in ihrem Bezug zu den Gelenkpositionen eine wesentliche Rolle.

Die GB-A-21 21 688 offenbart ein schwerpunktgesteuertes Anzeigesystem zur Vermessung von Unterschenkelprothesen. Für jeden Fuß der zu vermessenden Person ist jeweils eine Druckplatte vorgesehen, wobei für jeden der beiden Füße des Patienten der Schwerpunkt gesondert berechnet wird. Dabei sind die Druckplatten mit über eine Schaltung miteinander verbundenen Drucksensoren bestückt, die ein die ermittelte Schwerpunktlage definierendes elektrisches Signal abgeben. Die ermittelte Schwerpunktlage führt zum Aufleuchten einer Lampe in einer dicht neben dem zu vermessenden Patienten angeordneten Lichterreihe, wobei die aufleuchtende Lampe die dem gemessenen Schwerpunkt zukommende Frontal-Ebene markiert. Zur Visualisierung einer lotrechten Meßlinie muß dann von Hand ein lotrechter Stab horizontal bis neben bzw. vor die aufleuchtende Lampe verschoben werden. Anstelle dieses Stabes kann auch eine lotrechte Lichtquelle Verwendung finden. Da sich hierdurch jedoch keine lotrechte Meßlinie am Körper des zu vermessenden Patienten darstellen ließe, müßte der genannte lotrechte Stab durch einen lotrechten Lichtstab ersetzt werden, der in gleicher Weise in die optisch angezeigte Position zu verschieben ist und sich dann klar gegenüber dem Bein des Patienten optisch abhebt.

US-A-4,832,049 offenbart ein Anzeigesystem zur Vermessung einer Abnormität der Wirbelsäule, wobei auf den Rücken des Patienten Laser-Markierungen erzeugt werden. Die NL-A-74 15 910 offenbart eine Röntgeneinrichtung zur Durchführung von Aufnahmen einer aufrecht stehenden Person. In der XP-000267544 (Clin.Biomeh. 1992,7, 80-86) sind eine Vorrichtung und ein Verfahren zur Bestimmung der Relativposition bestimmter Wirbel gegenüber der Schwerkraftlinie des gesamten Körpers beschrieben. Auch die EP-A-0 119 660 A1 offenbart ein Vermessungssystem des Knochengerüstes unter Verwendung einer Meßplatte mit über eine Schaltung miteinander verbundenen Drucksensoren zur Ermittlung der Gewichtsverteilung einer auf der Meßplatte stehenden Person.

Der Erfindung liegt die Aufgabe zugrunde, ein Anzeigesystem zu entwickeln, mit dem sich die Körperschwerkraftlinie einfach, schnell und exakt visuell darstellen läßt.

Diese Aufgabe wird erfindungsgemäß gelöst durch ein Anzeigesystem, bestehend aus einer einteilig ausgebildeten Meßplatte zur Ermittlung des in die Ebene dieser Meßplatte projizierten Schwerpunktes einer auf der Meßplatte stehenden Person, aus einem Antrieb und aus einer Projektionseinrichtung zur Visualierung einer lotrechten Meßlinie auf den Körper dieser Person, wobei die Projektionseinrichtung ein optisches System aufweist und wobei die Meßplatte mit über eine Schaltung miteinander verbundenen Drucksensoren bestückt ist, die ein die ermittelte Schwerpunktlage definierendes elektrisches Steuersignal abgeben, mit dem der Antrieb gesteuert wird, der in einer Ebene parallel zu der der Meßplatte des außerhalb dieser Meßplatte angeordnete optische System verschiebt, das einen Lichtstrahl als lotrechte Meßlinie projiziert, die eine Ebene definiert, in der der Schwerpunkt der vermessenen Person liegt.

Mit diesem Anzeigesystem läßt sich die vertikale Körperschwerkraftlinie in der sagittalen und/oder frontalen Ebene auf den Körper projizieren, wobei im Ausgangspunkt der Vermessung diese Schwerpunktlinie genau in der durch den Körperschwerpunkt gehenden Ebene liegt. Bei der entsprechenden Ausgangsstellung folgt der Lichtstrahl den Bewegungen des Körperschwerpunktes mit einer Genauigkeit von ≤ ± 2 mm.

Neben dieser Grundprojektion ermöglicht das Anzeigesystem auch Abstandsmessungen zwischen dieser Schwerkraftlinie und besonderen Punkten auf dem zu vermessenden Körper oder auf einem prothetischen oder orthetischen Hilfsmittel (z.B. die Position eines Gelenkes zur Körperschwerachse).

Grundsätzlich ist es ferner möglich, über die Meßplatte auch das Gewicht der zu vermessenden Person anzuzeigen.

Erfindungsgemäß ist es vorteilhaft, wenn der Antrieb ein Schrittmotor ist. Um in der Projektionseinrichtung nur geringe Massen bewegen zu müssen, ist es dabei zweckmäßig, wenn der Schrittmotor - bezogen auf das Anzeigesystem - ortsfest angeordnet ist. Das erfindungsgemäße Anzeigesystem benötigt daher nur verhältnismäßig geringe elektrische Leistungen.

Eine konstruktiv einfache, präzise arbeitende Projektionseinrichtung ist dann gewährleistet, wenn der Schrittmotor einen endlosen Zahnriemen antreibt, der mit einem das optische System tragenden Schlitten verbunden ist. Dabei ergibt sich eine im Aufbau besonders einfache Konstruktion für die Projektionseinrichtung dann, wenn die Projektionseinrichtung eine gerade Schlittenführung aufweist, an deren beiden Enden je ein Gehäuse vorgesehen ist, von dem das eine Gehäuse den Schrittmotor mit dem Antriebsrad für den Zahnriemen und das andere Gehäuse das Gegenlager für die Umlenkung des Zahnriemens aufnehmen.

Der Lichtstrahl kann vorzugsweise ein Laserstrahl sein.

Bei einer ersten Alternativlösung ist es vorteilhaft, wenn der den Laserstrahl sendende Laser - bezogen auf das Anzeigesystem - ortsfest angeordnet ist und das optische System einen linear geführten Umlenkspiegel aufweist. Dabei ist der Umlenkspiegel vorzugsweise auf einem Schlitten montiert, der auf einer Schlittenführung geführt ist. Um mit dem umgelenkten Laserstrahl eine lotrechte Meßlinie auf dem zu vermessenden Körper projizieren zu können, ist es vorteilhaft, wenn der Umlenkspiegel um eine parallel zu seiner Verschiebungsrichtung liegende Achse drehbar ist. Zweckmäßig ist hierfür ein auf dem Schlitten montierter Motor vorgesehen, auf dessen Antriebswelle der Umlenkspiegel befestigt ist.

In einer zweiten Alternativlösung ist erfindungsgemäß vorgesehen, daß die den Lichtstrahl aussendende Lichtquelle an einem Schlitten montiert ist, der auf einer Schlittenführung geführt ist. Dabei ist die Lichtquelle vorzugsweise ein Laser mit Strichoptik. Bei dieser Konstruktion kann auf einen Umlenkspiegel mit seinem Antrieb verzichtet werden.

Als Trittmaterial für die Meßplatte wird erfindungsgemäß vorzugsweise ein Honeycomb-Material vorgesehen. Insbesondere aufgrund der sich hieraus ergebenden Flexibilität ist es möglich, die Meßplatte erfindungsgemäß einteilig auszubilden.

Weitere Merkmale der Erfindung sind Gegenstand der Unteransprüche und werden mit weiteren Vorteilen der Erfindung anhand eines Ausführungsbeispieles näher erläutert.

In der Zeichnung ist eine als Beispiel dienende Ausführungsform der Erfindung schematisch dargestellt. Es zeigen:
- Figur 1: in Draufsicht ein aus einer Projektionseinrichtung und einer Meßplatte bestehendes Anzeigesystem;

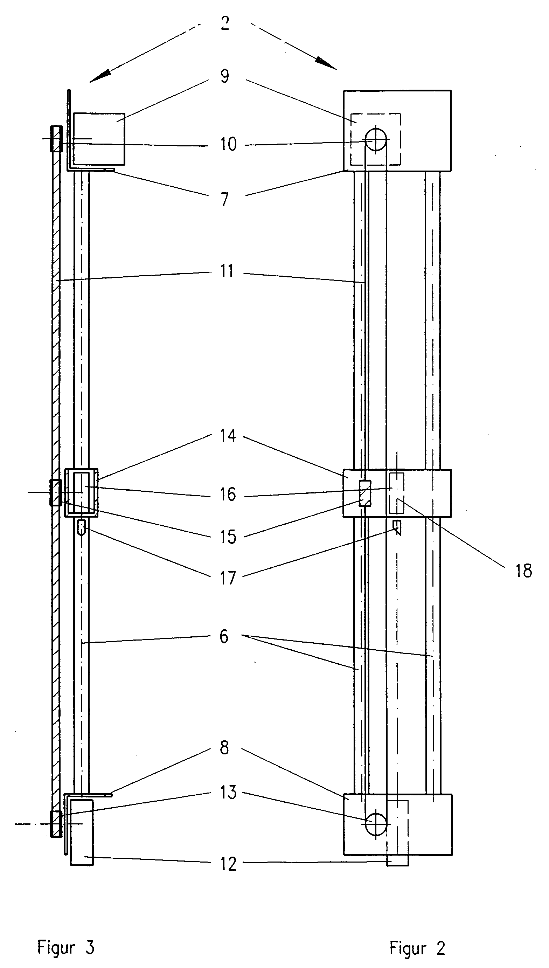

- Figur 2: in vergrößertem Maßstab in Draufsicht die Projektionseinrichtung und
- Figur 3: die Darstellung gemäß Figur 2 in Stirnansicht und teilweise im Querschnitt.

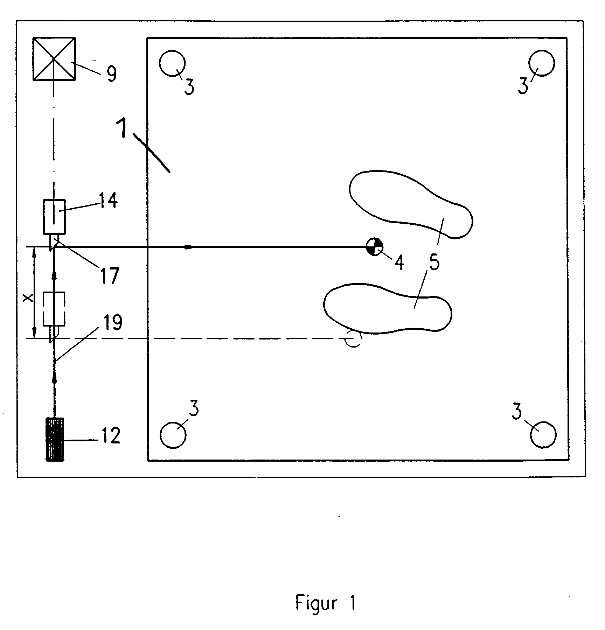

Das in Figur 1 dargestellte, stark schematisierte Anzeigesystem umfaßt eine Meßplatte 1 sowie eine Projektionseinrichtung 2, die vorzugsweise zu einer Einheit miteinander verbunden sind. Dadurch liegt die Justierung der Projektionseinrichtung 2 gegenüber der Meßplatte 1 unveränderlich fest; außerdem läßt sich das Anzeigesystem sehr einfach handhaben.

Die Meßplatte 1 ist einteilig ausgebildet, besteht im wesentlichen aus Honeycomb-Material und weist zumindest vier, vorzugsweise in den Ecken der Meßplatte 1 angeordnete Drucksensoren 3 auf, bei denen es sich um Kraftmeßdosen handelt, die über eine in der Zeichnung nicht näher dargestellte Schaltung miteinander verbunden sind. Mit Hilfe dieser Sensorschaltung läßt sich der Schwerpunkt 4 einer auf der Meßplatte 1 stehenden Person (von der in Figur 1 nur die beiden Füße 5 schematisch angedeutet sind) in seiner Lage innerhalb der durch die Meßplatte 1 gebildeten Ebene ermitteln.

Die Projektionseinrichtung 2 besteht im wesentlichen aus einer geraden Schlittenführung 6, an deren beiden Enden je ein Gehäuse 7, 8 vorgesehen ist. Das eine Gehäuse 7 beherbergt einen Schrittmotor 9 mit einem Antriebsrad 10 für einen endlosen Zahnriemen 11, während das andere Gehäuse 8 einen Laser 12 sowie das Gegenlager 13 für die Umlenkung des Zahnriemens 11 aufnimmt.

Auf der Schlittenführung 6 ist ein Schlitten 14 verschiebbar gelagert, der über eine Klemmeinrichtung 15 oder dergl. mit dem Zahnriemen 11 koppelbar ist und einen Motor 16 trägt, auf dessen Antriebswelle ein Umlenkspiegel 17 befestigt ist. Der Motor 16 ist so angeordnet, daß der Umlenkspiegel 17 parallel zu der Verschiebungsrichtung des Schlittens 14 bzw. parallel zur Schlittenführung 6 liegende Achse 18 drehbar ist.

Der Laser 12 sendet einen Lichtstrahl 19 aus, der - wie Figur 1 erkennen läßt - auf den Umlenkspiegel 17 trifft und von diesem rechtwinklig umgelenkt wird in Richtung der Meßplatte 1. Durch Rotation des Umlenkspiegels 17 läßt sich mit dem Lichtstrahl 19 eine lotrechte Ebene aufspannen. Da aber letztlich nur die linienförmige Projektion dieser Ebene auf der zu vermessenden Person benötigt wird, ist es vorteilhaft, für den Umlenkspiegel 17 einen reversiblen Drehantrieb mit einem Drehwinkel von z.B. nur 60° vorzusehen.

Die Drucksensoren 3, die die Lage des Schwerpunktes 4 einer zu vermessenden, auf der Meßplatte 1 stehenden Person ermitteln, geben ein elektrisches Signal ab, das der Lage des Schwerpunktes 4 auf der in Figur 1 eingezeichneten x-Achse entspricht. Mit diesem elektrischen Signal wird der Schrittmotor 9 beaufschlagt, der daraufhin über den Zahnriemen 11 den Schlitten 14 in diejenige x-Position verschiebt, in der der umgelenkte Lichtstrahl 19 bzw. die mit ihm definierte lotrechte Ebene die gleiche x-Position einnehmen wie der ermittelte Schwerpunkt 4, der somit in der durch den Lichtstrahl 19 aufgespannten Ebene liegt, die sich als lotrechte Meßlinie auf der zu vermessenden Person darstellt. Um nun den exakten x-Abstand eines Gelenkpunktes, Paßteils oder dergl. von dieser Schwerpunktebene ermitteln zu können, wird durch manuelle Steuerung des Schrittmotors 9 der Schlitten 14 und damit die vom Umlenkspiegel 17 erzeugte lotrechte Meßlinie an der zu vermessenden Person so lange um eine Stecke x₁ verschoben, bis diese lotrechte Meßlinie den zu vermessenden Punkt schneidet. Die Wegstrecke x₁ kann beispielsweise digital angezeigt werden, so daß sich die jeweiligen Abstandsmessungen von nur einer Bedienungsperson schnell, unkompliziert und exakt durchführen lassen.

## Patentansprüche

1. Schwerpunktgesteuertes Anzeigesystem zur Vermessung des menschlichen Körpers und/oder prothetischer und orthetischer Paßteile in ihrer relativen Position zum menschlichen Körper, bestehend aus einer einteilig ausgebildeten Meßplatte (1) zur Ermittlung des in die Ebene dieser Meßplatte (1) projizierten Schwerpunktes (4) einer auf der Meßplatte (1) stehenden Person, aus einem Antrieb und aus einer Projektionseinrichtung (2) zur Visualisierung einer lotrechten Meßlinie auf den Körper dieser Person, wobei die Projektionseinrichtung ein optisches System aufweist wobei die Meßplatte (1) mit über eine Schaltung miteinander verbundenen Drucksensoren (3) bestückt ist, die ein die ermittelte Schwerpunktlage definierendes elektrisches Steuersignal abgeben, mit dem der Antrieb gesteuert wird, der in einer Ebene parallel zu der der Meßplatte (1) das außerhalb dieser Meßplatte (1) angeordnete optische System verschiebt, das einen Lichtstrahl (19) als lotrechte Meßlinie projiziert, die eine Ebene definiert, in der der Schwerpunkt (4) der vermessenen Person liegt.

2. Anzeigesystem nach Anspruch 1, **dadurch gekennzeichnet,** daß der Antrieb ein Schrittmotor (9) ist.

3. Anzeigesystem nach Anspruch 2, **dadurch gekennzeichnet,** daß der Schrittmotor (9) - bezogen auf das Anzeigesystem - ortsfest angeordnet ist.

4. Anzeigesystem nach Anspruch 3, **dadurch gekennzeichnet,** daß der Schrittmotor (9) einen endlosen Zahnriemen (11) antreibt, der mit einem das optische System tragenden Schlitten (14) verbunden ist.

5. Anzeigesystem nach einem der vorhergehenden Ansprüche, **dadurch gekennzeichnet,** daß die Projektionseinrichtung (2) eine gerade Schlittenführung (6) aufweist, an deren beiden Enden je ein Gehäuse (7, 8) vorgesehen ist, von dem das eine Gehäuse (7) den Schrittmotor (9) mit dem Antriebsrad (10) für den Zahnriemen (11) und das andere Gehäuse (8) das Gegenlager (13) für die Umlenkung des Zahnriemens (11) aufnehmen.

6. Anzeigesystem nach einem der vorhergehenden Ansprüche, **dadurch gekennzeichnet,** daß das optische System aus seiner genannten Projektionsposition durch manuelle Steuerung um jeweils definierte Strecken (x) verfahrbar ist.

7. Anzeigesystem nach einem der vorhergehenden Ansprüche, **dadurch gekennzeichnet,** daß der Lichtstrahl (19) ein Laserstrahl ist.

8. Anzeigesystem nach Anspruch 7, **dadurch gekennzeichnet,** daß der den Laserstrahl sendende Laser (12) - bezogen auf das Anzeigesystem - ortsfest angeordnet ist, und das optische System einen linear geführten Umlenkspiegel (17) aufweist.

9. Anzeigesystem nach Anspruch 8, **dadurch gekennzeichnet,** daß der Umlenkspiegel (17) auf einem Schlitten (14) montiert ist, der auf einer Schlittenführung (6) geführt ist.

10. Anzeigesystem nach Anspruch 8 oder 9, **dadurch gekennzeichnet,** daß der Umlenkspiegel (17) um eine parallel zu seiner Verschiebungsrichtung liegende Achse (18) drehbar ist.

11. Anzeigesystem nach Anspruch 10, **gekennzeichnet** durch einen motorischen Antrieb (16) für die Drehung des Umlenkspiegels (17).

12. Anzeigesystem nach Anspruch 11, **dadurch gekennzeichnet,** daß der Schlitten (14) einen Motor (16) trägt, auf dessen Antriebswelle der Umlenkspiegel (17) befestigt ist.

13. Anzeigesystem nach einem der Ansprüche 1 bis 7, **dadurch gekennzeichnet,** daß die den Lichtstrahl (19) aussendende Lichtquelle an einem Schlitten (14) montiert ist, der auf einer Schlittenführung (6) geführt ist.

14. Anzeigesystem nach Anspruch 13, **dadurch gekennzeichnet,** daß die Lichtquelle ein Laser mit Strichoptik ist.

15. Anzeigesystem nach einem der vorhergehenden Ansprüche, **dadurch gekennzeichnet,** daß die Meßplatte (1) einteilig ausgebildet ist.

16. Anzeigesystem nach Anspruch 15, **dadurch gekennzeichnet,** daß die Meßplatte (1) im wesentlichen aus Honeycomb-Material besteht.

17. Anzeigesystem nach einem der vorhergehenden Ansprüche, **dadurch gekennzeichnet,** daß die Projektionseinrichtung (2) und die Meßplatte (1) zu einer Einheit miteinander verbunden sind.

## Claims

1. Centre-of-gravity-controlled indicating system for measuring the human body and/or prosthetic and orthotic fitting parts in their relative position to the human body, comprising a measuring plate (1), formed as a single piece, for finding the centre of gravity (4), projected in the plane of said measuring plate (1), of an individual standing on the measuring plate (1), a drive and a projection device (2) for visualizing a perpendicular measurement line on the body of said individual, the projection device having an optical system and the measuring plate (1) being fitted with pressure sensors (3) which are interconnected by means of a circuit and which emit an electrical control signal which defines the position of the centre of gravity found and with which the drive is controlled which displaces the optical system disposed outside said measuring plate (1) in a plane parallel to that of the measuring plate (1), which optical system projects a light beam (19) as a perpendicular measurement line which defines a plane in which the centre of gravity (4) of the individual measured is situated.

2. Indicating system according to Claim 1, characterized in that the drive is a stepping motor (9).

3. Indicating system according to Claim 2, characterized in that the stepping motor (9) is disposed in a stationary manner relative to the indicating system.

4. Indicating system according to Claim 3, characterized in that the stepping motor (9) drives a continuous toothed belt (11) which is connected to a carriage (14) supporting the optical system.

5. Indicating system according to one of the preceding claims, characterized in that the projection device (2) has a straight carriage guide (6) at whose two ends a housing (7, 8) is provided in each case, of which one housing (7) accommodates the stepping motor (9) with the drive wheel (10) for the toothed belt (11) and the other housing (8) accommodates the counterbearing (13) for the return of the toothed belt (11).

6. Indicating system according to one of the preceding claims, characterized in that the optical system can be moved from its said projection position by manual control over defined distances (x) in each case.

7. Indicating system according to one of the preceding claims, characterized in that the light beam (19) is a laser beam.

8. Indicating system according to Claim 7, characterized in that the laser (12) emitting the laser beam is disposed in a stationary manner relative to the indicating system and the optical system has a linearly guided path-folding mirror (17).

9. Indicating system according to Claim 8, characterized in that the path-folding mirror (17) is mounted on a carriage (14) which is guided on a carriage guide (6).

10. Indicating system according to Claim 8 or 9, characterized in that the path-folding mirror (17) is rotatable about an axis (18) situated parallel to its direction of displacement.

11. Indicating system according to Claim 10, characterized by a motor drive (16) for the rotation of the path-folding mirror (17).

12. Indicating system according to Claim 11, characterized in that the carriage (14) supports a motor (16) to whose drive shaft the path-folding mirror (17) is attached.

13. Indicating system according to one of Claims 1 to 7, characterized in that the light source emitting the light beam (19) is mounted on a carriage (14) which is guided on a carriage guide (6).

14. Indicating system according to Claim 13, characterized in that the light source is a laser with reticle optics.

15. Indicating system according to one of the preceding claims, characterized in that the measuring plate (1) is formed as a single piece.

16. Indicating system according to Claim 15, characterized in that the measuring plate (1) substantially comprises honeycomb material.

17. Indicating system according to one of the preceding claims, characterized in that the projection device (2) and the measuring plate (1) are interconnected to form a unit.

## Revendications

1. Système d'affichage asservi au centre de gravité, pour la mensuration du corps humain et/ou de pièces adaptant des prothèses ou orthèses dans leur positionnement par rapport au corps humain, constitué d'un plateau de mesure (1) pour la détermination du centre de gravité (4) projeté dans le plan de ce plateau de mesure (1) d'une personne se tenant sur le plateau de mesure (1), d'un entraînement et d'un dispositif de projection (2) pour la visualisation sur le corps de cette personne d'une ligne verticale de mesure, le dispositif de mesure présentant un système optique, le plateau de mesure (1) étant équipé de détecteurs de pression (3) reliés les uns aux autres par l'intermédiaire d'un circuit et qui émettent un signal électrique définissant la position détectée du point de mesure, par lequel est commandé l'entraînement qui déplace dans un plan parallèle à celui du plateau de mesure (1) le système optique disposé à l'extérieur du plateau de mesure (1) et projetant en tant que ligne verticale de mesure un rayon lumineux (19) qui définit un plan dans lequel se trouve le centre de gravité (4) de la personne dont la mensuration est effectuée.

2. Système d'affichage selon la revendication 1, caractérisé en ce que l'entraînement est un moteur pas-à-pas (9).

3. Système d'affichage selon la revendication 2, caractérisé en ce que le moteur pas-à-pas (9) est disposé de manière stationnaire par rapport au système d'affichage.

4. Système d'affichage selon la revendication 3, caractérisé en ce que le moteur pas-à-pas (9) entraîne une courroie crantée sans fin (11) qui est reliée à un chariot (14) portant le système optique.

5. Système d'affichage selon l'une quelconque des revendications précédentes, caractérisé en ce que le dispositif de projection (2) présente un guide rectiligne de chariot (6) à chacune des deux extrémités duquel est prévu un boîtier (7, 8) dont un boîtier (7) reçoit le moteur pas-à-pas (9) avec la roue d'entraînement (10) pour la courroie crantée (11), le second boîtier (8) recevant le contre-palier (13) pour le renvoi de la courroie crantée (11).

6. Système d'affichage selon l'une quelconque des revendications précédentes, caractérisé en ce que le système optique peut être déplacé par commande manuelle hors de sa dite position de projection, chaque fois d'une distance définie (x).

7. Système d'affichage selon l'une quelconque des revendications précédentes, caractérisé en ce que le rayon lumineux (19) est un rayon laser.

8. Système d'affichage selon la revendication 7, caractérisé en ce que le laser (12) émettant le rayon laser est stationnaire par rapport au système d'affichage, le système optique présentant un miroir de déviation (17) guidé en ligne droite.

9. Système d'affichage selon la revendication 8, caractérisé en ce que le miroir de déviation (17) est monté sur un chariot (14) qui est guidé sur un guide de chariot (6).

10. Système d'affichage selon la revendication 8 ou 9, caractérisé en ce que le miroir de déviation (17) peut tourner autour d'un axe (18) parallèle à la direction de son déplacement.

11. Système d'affichage selon la revendication 10, caractérisé par un entraînement à moteur (16) pour la rotation du miroir de déviation (17).

12. Système d'affichage selon la revendication 11, caractérisé en ce que le chariot (14) porte un moteur (16) sur l'arbre d'entraînement duquel est fixé le miroir de déviation (17).

13. Système d'affichage selon l'une quelconque des revendications 1 à 7, caractérisé en ce que la source lumineuse émettant le rayon lumineux (19) est montée sur un chariot (14) qui est guidé sur un guide de chariot (6).

14. Système d'affichage selon la revendication 13, caractérisé en ce que la source lumineuse est un laser avec optique à grille.

15. Système d'affichage selon l'une quelconque des revendications précédentes, caractérisé en ce que le plateau de mesure (1) est réalisé en une seule pièce.

16. Système d'affichage selon la revendication 15, caractérisé en ce que le plateau de mesure (1) est essentiellement constitué d'un matériau en nid d'abeilles.

17. Système d'affichage selon l'une quelconque des revendications précédentes, caractérisé en ce que la direction de projection (2) et le plateau de mesure (1) sont reliés l'un à l'autre de manière à former une unité.
